(19) Europäisches Patentamt
European Patent Office
Office européen des brevets

(11) Veröffentlichungsnummer: **0 003 989**
**B1**

(12) # EUROPÄISCHE PATENTSCHRIFT

(21) Anmeldenummer: **79100490.6**

(51) Int. Cl.³: **C 07 C 125/06**

(22) Anmeldetag: **20.02.79**

(54) **Verfahren zur Herstellung von Urethanen**

(30) Priorität: **02.03.78 DE 2808980**

(43) Veröffentlichungstag der Anmeldung:
**19.09.79 Patentblatt 79/19**

(45) Bekanntmachung des Hinweises auf die Patenterteilung:
**10.12.80 Patentblatt 80/25**

(84) Benannte Vertragsstaaten:
**BE DE FR GB IT NL**

(56) Entgegenhaltungen:
**DE - A - 2 343 826**
**DE - A - 2 614 101**
**DE - A - 2 623 694**
**DE - B - 2 555 557**

(73) Patentinhaber: **BAYER AG**
**Zentralbereich Patente, Marken und Lizenzen**
**D - 5090 Leverkusen 1, Bayerwerk (DE)**

(72) Erfinder: **Scholl, Hans-Joachim, Dr.**
**Rudolf-Sohm-Strasse 28**
**D - 5000 Köln 60 (DE)**
**Zenner, Armin, Dr.**
**Goethestrasse 65**
**D - 4047 Dormagen 1 (DE)**

**EP 0 003 989 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von Urethanen

Die vorliegende Erfindung betrifft ein verbessertes Verfahren zur Herstellung von Urethanen durch Umsetzung von aromatischen Nitroverbindungen mit Alkoholen und Kohlenmonoxid in Gegenwart von Selen enthaltenden Katalysatorsystemen. Aus den genannten Ausgangsmaterialien zugängliche Urethane wurden bischer im allgemeinen durch Umsetzung eines aromatischen Isocyanats mit einem Alkohol gebildet, wobei das Isocyanat seinerseits durch Umsetzung von Phosgen mit dem entsprechenden primären Amin gewonnen wird, welches in allgemeinen wiederum durch Reduktion der entsprechenden Nitroverbindung erhalten worden war. Dieses herkömmliche Verfahren weist jedoch verschiedene Nachteile auf, nicht zuletzt aufgrund der Toxizität und der korrosiven Natur von Phosgen und der Bildung von Chlorwasserstoff als Nebenprodukt. Außerdem ist es bekannt, daß bestimmte aromatische Amine schädliche biologische Eigenschaften aufweisen und einige auch dazu neigen, bei der Lagerung durch Luft oxydiert zu werden.

Es mangelte daher auch nicht an Versuchen, den Umweg über das hochtoxische Phosgen zu vermeiden und die Urethane direkt aus den entsprechenden Nitroverbindungen und den entsprechenden Alkoholen sowie Kohlenmonoxid herzustellen. Die Verfahren der US—PS 3 993 685 bzw. der DT—OS 2 603 574 bedienten sich hierbei Katalysatorsysteme auf Basis von Metallen der Platin-Gruppe. Da bei diesen Verfahren merkliche Verluste der sehr kostspieligen Katalysatoren nicht vermieden werden können, fanden diese Verfahren bislang keinen Eingang in die großtechnische Praxis.

Beim Verfahren der DT—OS 2 343 826 wird als katalytisch wirkendes System eine Kombination aus Selen oder Schwefel bzw. Verbindungen dieser Elemente mit sehr großen Mengen einer Base vorgeschlagen. Als Basen kommen z.B. Triäthylamin und Pyridin in Frage. Um aber die Reaktion in Anwesenheit dieser Amine zufriedenstellend einzuleiten, erscheint es erforderlich, die tertiären Amine in einer ziemlich großen Menge im Hinblick auf die Ausgangsnitroverbindung zu verwenden, In der Tat wird, wenn Dinitrotoluol als Nitroverbindung verwendet wird, das tertiäre Amin in einer Menge verwendet, die gleich oder größer ist als diejenige des Dinitrotoluols. Die Verwendung derart großer Mengen an tertiärem Amin bringt zahlreiche Probleme wirtschaftlicher Art und auch hinsichtlich der Rückgewinnungsverfahren mit sich. Weiterhin führt dieses Verfahren zur Bildung von Nebenprodukten, z.B. Aminoverbindungen und Harnstoffen, wenn meßbare Anteile an Wasser, z.B. als Hydrate, aber auch in freier Form vorliegen. Auch das Verfahren der DT—OS 2 343 826 ist daher für eine großtechnische Anwendung weitgehend ungeeignet.

Die wegen der gennanten Bildung von Nebenprodukten herabgesetzte Ausbeute an den angestrebten Urethanen läßt sich beim Verfahren der DT—OS 2 614 101 durch ein Katalysator-System vermeiden, welches aus elementarem Selen bzw. einer Selenverbindung und einem Promotor, bestehend z.B. aus einem bicyclischen Amidin und einer Carbonsäure zusammengesetzt ist. Zwar sind beim Verfahren der DT—OS 2 614 101 die Ausbeuten an Urethanen im Vergleich zum Verfahren der DT—OS 2 343 826 erhöht, jedoch entstehen auch hierbei störende Mengen an Nebenprodukten, welche insbesondere Hydrolyse- und Folgeprodukte des gebildeten Urethans darstellen.

Das Verfahren der DT—OS 2 623 694 muß insofern als eine Weiterentwicklung des Verfahrens der DT—OS 2 614 101 betrachtet werden, als die Bildung der Nebenprodukte aus den Urethanen durch Mitverwendung von diesen Nebenprodukten entsprechenden aromatischen Aminoverbindungen bzw. aromatischen Harnstoffverbindungen zurückgedrängt wird. Obwohl mit dieser Maßnahme eine Verbesserung des Verfahrens gemäß DT—OS 2 614 101 möglich ist, ist auch das Verfahren der DT—OS 2 623 694 noch mit schwerwiegenden Mängeln behaftet. Insbesondere ist bei diesem Verfahren die Verwendung von ungewöhnlich hohen Mengen an Selen bzw. Selenverbindungen erforderlich, was zu erheblichen Verlusten dieses Katalysators führt. Darüberhinaus handelt es sich bei Selen bzw. den einzusetzenden Selenverbindungen um toxikologisch bedenkliche Substanzen, die darüberhinaus dem hergestellten Urethan einen unangenehmen Geruch verleihen.

Es war daher Aufgabe der vorliegende Erfindung, ein verbessertes Verfahren zur Herstellung von Urethanen aus aromatischen Nitroverbindungen, Alkoholen und Kohlenmonoxid zur Verfügung zu stellen, bei welchem die Mange des Selens bzw. der Selenverbindung ganz wesentlich reduziert werden kann, wobei trotz der Reduzierung der Katalysatorenmenge eine möglichst quantitative Urethanbildung ermöglich wird.

Diese Aufgabe konnte überaschenderweise durch das nachstehend näher erläuterte erfindungsgemäße Verfahren gelöst werden, wobei die erfindungsgemäß einzusetzende Menge an Selen bzw. Selenverbindung so drastisch reduziert werden konnte, daß die geschilderten Reinigungs-, Toxikologie- und Abtrennungsprobleme weitgehend entfallen.

Gegenstand der vorliegenden Erfindung ist ein Verfahren zur Verfahren zur Herstellung von Urethanen durch Umsetzung von aromatischen Nitroverbindungen mit aliphatischen, cycloaliphatischen oder araliphatischen Alkoholen und Kohlenmonoxid in Gegenwart von

Selen und/oder Selenverbindungen,
aromatische Aminoverbindungen und/oder aromatische Harnstoffverbindungen, sowie
Alkalisalze schwacher Säuren und/oder bicyclische Amidine der Formel

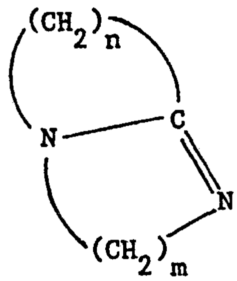

in welcher
n für eine ganze Zahl von 3 bis 5 und
m für eine ganze Zahl von 2 bis 4
stehen oder Alkalisalze schwacher Säuren in Kombination mit sonstigen tertiären Aminen mit 3 bis 20 Kohlenstoffatomen
aufweisenden Katalysator-Systemen, dadurch gekennzeichnet, daß man Katalysator-Systeme verwendet, welche Oxydationsmittel ausgewählt aus der Gruppe bestehend aus Sauerstoff, oxydierend wirkenden, chemisch gebundenen Sauerstoff enthaltenden organischen Verbindungen und oxydierend wirkenden, chemisch gebundenen Saurestoff enthaltenden anorganischen Verbindungen von Metallen der 1,2,5—8 Nebengruppe des Periodensystems der Elemente enthalten.

Ausgangsverbindungen für das erfindungsgemäße Verfahren sind

1. aromatische Nitroverbindungen, wie z.B. Nitrobenzol, 1,3-Dinitrobenzol, o-Nitrotoluol, m-Nitrotoluol, p-Nitrotoluol, 2,4-Dinitrotoluol, 2,6-Dinitrotoluol, Nitronaphthaline, Nitroanthracene, Nitrobiphenylene u.dgl. Im allgemeinen weisen die erfindungsgemäß geeigneten Nitroverbindungen ein Molekulargewicht von 123—400, 1—3 aromatische Kerne, sowie 1—3 an aromatische Kerne gebundene Nitrogruppen, sowie gegebenenfalls weitere unter den Reaktionsbedingungen des erfindungsgemäßen Verfahrens inert Substituenten auf. Zu den bevorzugten Nitroverbindungen für das erfindungsgemäße Verfahren gehören Nitrobenzol, sowie die genannten Dinitrotoluole. Beliebige Gemische der gennanten Nitroverbindungen können selbstverständlich ebenfalls eingesetzt werden.

2. Aliphatische, cyclialiphatische oder araliphatische Alkohole, d.h. vorzugsweise beliebige, unter den Reaktionsbedingungen ansonsten inerte organische Verbindungen mit mindestens einer aliphatisch, cycloaliphatisch oder araliphatisch gebundenen Hydroxygruppe des Molekulargewichtbereichs 32—300. Beispiele geeigneter Alkohole sind primäre, sekundäre oder tertiäre Alkohole wie z.B. Methanol, Äthanol, n-Propanol, iso-Propanol, die verschiedenen isomeren Butanole, Cyclohexylalkohol, Benzylalkohol, Hexylalkohol, Laurylalkohol, Cetylalkohol u.dgl. Vorzugsweise werden beim erfindungsgemäßen Verfahren einwertige Alkohole, besonders bevorzugt Äthanol, eingesetzt.

3. Gasförmiges Kohlenmonoxid Beim erfindungsgemäßen Verfahren werden Katalysatoren-Systeme eingesetzt, welche a) Selen bzw. eine Selenverbindung, b) ein Oxidationsmittel, c) ein bicyclisches Amidin und/oder ein Alkalisalz einer schwachen Säure und/oder Kombinationen derartiger Salze mit sonstigen teriären Aminen mit 3 bis 20 C-Atomen und d) eine aromatische Aminoverbindung und/oder eine aromatische Harnstoffverbindung aufweisen.

Geeignete Katalysatoren-Komponenten a) sind entweder elementares Selen in beliebiger Form, vorzugsweise metallisches Selen, oder anorganische Selenverbindungen wie z.B. Selendioxid oder Carbonylselenid (COSe). Prinzipiell denkbar ist auch die Verwendung von organischen Selenverbindungen, wie z.B. Dimethylselenid, Diphenylselenid u.dgl. Elementares Selen ist besonders bevorzugt.

Bei den Oxidationsmitteln b) handelt es sich entweder um elementaren Sauerstoff bzw. ein Sauerstoff enthaltendes Gas wie z.B. Luft und/oder um oxidierend wirkende, chemisch gebundenen Sauerstoff aufweisende organische Verbindungen wie z.B. Chinone vorzugsweise 1,4-Benzochinon und/oder um oxidierend wirkende chemisch gebundenen Sauerstoff aufweisende anorganische Verbindungen von Metallen. Bei den letztgenannten Verbindungen handelt es sich insbesondere um die entsprechenden Oxide. Es kommen vorzugsweise die entsprechenden Metallverbindungen der Elemente der 2, sowie 5—8 Nebengruppe des Periodensystems in Betracht. Besonders bevorzugt werden jedoch die entsprechenden Verbindungen der Elemente der 5 und 6 Nebengruppe sowie die entsprechenden Verbindungen von Mangan, Eisen, Kobalt und Nickel eingesetzt. Beispiele geeigneter Oxidationsmittel sind Zinkoxid, Eisen-II-oxid, Eisen-III-oxid, aus diesen letztgenannten Eisenoxiden bestehende Mischoxide, Vanadium-V-oxid, Mangan-IV-oxid, Molybdän-VI-oxid, Nickel-II-oxid, Kobalt-II-oxid, Mischoxide des 3- bis 6-wertigen Chroms, sowie beliebige Gemische der beispielhaft genannten Oxide. Zu den besonders bevorzugten Oxidationsmittel gehört Eisen-III-oxid. Ganz besonders bevorzugt sind Eisen, Vanadium und/oder Molybdän enthaltende Mischoxide.

Bei der Katalysatoren-Komponente c) handelt es sich um bicyclische Amidine der Formel

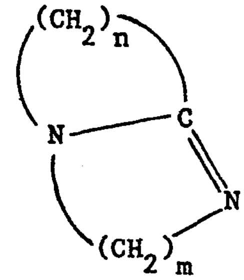

in welcher

n für eine ganze Zahl von 3 bis 5 und

m für eine ganze Zahl von 2 bis 4 stehen.

Neben oder anstelle der genannten bicyclischen Amidine können als Katalysator-Komponente c) auch basisch wirkende Alkalisalze schwacher Säuren insbesondere Alkalicarboxylate wie Natriumacetat, Kaliumacetat, Natriumbenzoat oder Alkalisalze schwacher anorganischer Säuren, wie z.B. Natriumborat oder Natriumcarbonat oder auch Kombinationen derartiger Salze mit tertiäre Aminogruppen aufweisenden organischen Basen wie z.B. tertiären aliphatischen Aminen mit insgesamt 3 bis 20 Kohlenstoffatomen wie Trimethylamin, Triäthylamin, N,N-Dimethyl-octadecylamin oder Trihexylamin, heterocyclische tertiäre Amine wie z.B. Pyridin oder 2 tert. Aminogruppen aufweisende Amine wie z.B. Diazabicyclo[2.2.2]-octan (Triäthylendiamin) verwendet werden. Zu den bevorzugten Katalysator-Komponenten c) gehören 1,5-Diazabicyclo[4.3.0]-non-5-en, 1,8-Diazabicyclo[5.4.0]-undecen-7 und Natrium- bzw. Kaliumacetat. Bevorzugt kommt weiterhin Triäthylendiamin, insbesondere in Kombination mit Salzen der Formel

MeX

zum Einsatz, wobei

Me für ein Alkalimetall-Kation und

X für ein Cyanat- oder Rhodanid-Anion stehen.

Bei Verwendung derartiger Kombinationen werden die letztgenannten Salze im allgemeinen in Mengen von 1 bis 40 Mol-% vorzugsweise von 4 bis 20 Mol-% bezogen auf die eingesetzte Nitroverbindung verwendet.

Bei der Katalysatoren-Komponente d) handelt es sich um beliebige aromatisch gebundene primäre Aminogruppen und/oder aromatisch gebundene Harnstoffgruppen aufweisende organische Verbindungen, die neben den genannten Gruppen, insbesondere auch noch Nitrogruppen und Urethangruppen aufweisen können. Im allgemeinen handelt es sich bei der Komponente d) der erfindungsgemäß einzusetzenden Katalysatoren-Systeme um Verbindungen bzw. Gemische von Verbindungen, welche den Formeln

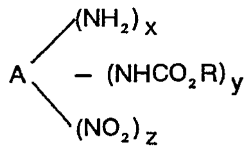

und / oder

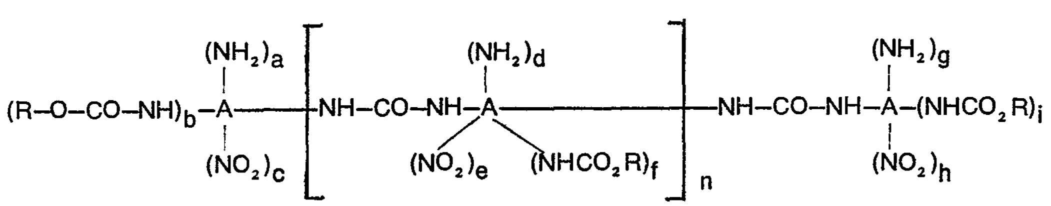

ensprechen.

In diesen Formeln bedeuten

x 1 oder 2,

y 0 oder 1,

z 0 oder 1, wobei die Summe x + y + z vorzugsweise 1 oder 1 2 beträgt,

a, b, c, d, e, f, g, h und i jeweils 0 oder 1, wobei die Summe a + b + c gleich der Summe g + h + i ist und 0, 1 oder 2 bedeutet, während die Summe d + e + f im Falle von a + b + c = 1 oder 2 einen um 1 verminderten Wert, d.h. 0 oder 1 darstellt, und im Fall von a + b + c = 0 ebenfalls für 0 steht,

n 0, 1, 2 oder 3, vorzugsweise jedoch 0 bedeutet,

A einen 1-, 2- oder 3-wertigen, vorzugsweise 1- oder 2-wertigen, gegebenenfalls $C_1$—$C_4$-Alkyl-substituierten aromatischen Kohlenwasserstoffrest, der im übrigen vorzugsweise dem aromatischen Kohlenwasserstoffrest der beim erfindungsgemäßen Verfahren einzusetzenden aromatischen Nitroverbindung entspricht und

R einen aliphatischen, cycloaliphatischen oder araliphatischen Kohlenwasserstoffrest mit im allgemeinen bis zu 18 Kohlenstoffatomen, der im übrigen vorzugsweise dem Kohlenwasserstoffrest der beim erfindungsgemäßen Verfahren einzusetzenden Alkoholkomponente entspricht.

Beispiele geeigneter Katalysatoren-Komponenten d) sind Anilin, o-, m- oder p-Toluidin,

die isomeren Nitroaniline, die isomeren Diaminobenzole, N,N'-Diphenylharnstoff, N,N'-Bis(2-methyl-5-nitro-phenyl)-harnstoff, N,N'-Bis(2-methyl - 5 - äthoxycarbonylamino - phenyl) - harnstoff, N,N' - Bis - (2 - methyl - 5 - amino - phenyl) - harnstoff, 2 - Amino - 4 - nitrotoluol, 4 - Amino - 2 - nitrotoluol, 2 - Amino - 4 - äthoxycarbonylamino - toluol, 4 - Amino - 2 - äthoxycarbonylamino - toluol, 2,4 - Diamino - toluol, N,N' - Bis - (3 - nitro - 4 - methyl - phenyl) - harnstoff, N,N' - Bis - (2 - methyl - 5 - nitrophenyl) - harnstoff, N,N' - Bis - (3 - äthoxycarbonylamino - 4 - methylphenyl) - harnstoff, N,N' - Bis - (2 - methyl - 5 - äthoxy - carbonylaminophenyl) - harnstoff, N,N' - Bis - (3 - amino - 4 - methylphenyl) - harnstoff, N,N' - Bis - (2 - methyl - 5 - aminophenyl) - harnstoff, N - (3 - Nitro - 4 - methyl - phenyl) - N' - (2 - methyl - 5 - nitrophenyl) - harnstoff, N - (3 - äthoxycarbonylamino - 4 - methylphenyl) - N' - (2 - methyl - 5 - äthoxycarbonylamino) - harnstoff, N - (3 - Amino - 4 - methylphenyl) - N' - (2 - methyl - 5 - aminophenyl) - harnstoff, N - (3 - Nitro - 4 - methyl - phenyl) - N' - (3 - äthoxycarbonylamino - 4 - methylphenyl) - harnstoff, N - (3 - Nitro - 4 - methylphenyl) - N' - (2 - methyl - 5 - äthoxycarbonylamino - phenyl) - harnstoff, N - (3 - Nitro - 4 - methyl - phenyl) - N' - (3 - amino - 4 - methyl - phenyl) - harnstoff, N - (3 - Nitro - 4 - methylphenyl) - N' - (2 - methyl - 5 - aminophenyl) - harnstoff, N - (2 - Methyl - 5 - nitrophenyl) - N' - (3 - äthoxycarbonylamino - 4 - methylphenyl) - harnstoff, N - (2 - Methyl - 5 - nitrophenyl) - N' - (2 - methyl - 5 - äthoxycarbonylamino - phenyl) - harnstoff, N - (2 - Methyl - 5 - nitro - phenyl) - N' - (3 - amino - 4 - methylphenyl) - harnstoff, N - (2 - Methyl - 5 - nitrophenyl) - N' - (2 - methyl - 5 - aminophenyl) - harnstoff, N - (3 - Äthoxycarbonylamino - 4 - methylphenyl) - N' - (2 - methyl - 5 - aminophenyl) - harnstoff, N - (2 - Methyl - 5 - äthoxycarbonylamino - phenyl) - N' - (3 - amino - 4 - methylphenyl) - harnstoff, N - (2 - Methyl - 5 - äthoxycarbonyl - aminophenyl) - N' - (2 - methyl - 5 - amino - phenyl) - harnstoff, sowie beliebige Gemische der beispielhaft genannten Verbindungen. Wie bereits dargelegt, werden vorzugsweise solche Verbindungen d) eingesetzt, die bezüglich ihres aromatischen Restes der beim erfindungsgemäßen Verfahren einzusetzenden aromatischen Nitroverbindung entsprechen. Bei Verwendung von Nitrobenzol wird demgemäß beispielsweise Anilin oder Diphenylharnstoff verwendet, während bei Verwendung von Nitrotoluol entweder ein Toluoylamin oder ein Ditoluoylharnstoff zum Einsatz gelangt. Dementsprechend werden bei Verwendung zweiwertiger Nitroverbindungen beispielsweise von 2,4-Dinitrotoluol, die entsprechenden, zweifach substituierte Toluoylrest aufweisenden Verbindungen eingesetzt.

Höhere Homologe der beispielhaft aufgeführten Harnstoffe, d.h. mehrere Harnstoffeinheiten aufweisende Verbindungen können ebenfalls eingesetzt werden.

Bei der Durchführung des erfindungsgemäßen Verfahrens kommen die Reaktionspartner im allgemeinen in solchen Mengen zum Einsatz, daß für jede Nitrogruppe der als Ausgangsmaterial eingesetzten aromatischen Nitroverbindung zwischen 1 bis 50 vorzugsweise zwischen 5 bis 30 Hydroxylgruppen der Alkoholkomponente vorliegen. Das Kohlenmonoxid wird im allgemeinen im Überschuß eingesetzt, da stets in Kohlenmonoxid-Atmosphäre gearbeitet wird; die gegebenenfalls den erfindungsgemäßen Anteil Sauerstoff aufweist.

Die Katalysatoren-Komponente a), d.h. das elementare Selen bzw. die Selenverbindung, welche auch auf einen geeigneten Träger, wie Kohlenstoff, Aluminiumoxid, Siliciumdioxid, Diatomeenerde, aktivierter Ton, Zeolith, Molekularsieben, Barium-sulfat, Kalziumcarbonat, Ionenaustauscherharzen und ähnlichen Materialien aufgebracht werden kann, wird in einer Menge eingesetzt, die 0,1 bis 10 Gew.-%, vorzugsweise 0,1 bis 3 Gew.-% Selen, bezogen auf die als Ausgangsmaterial eingesetzte Nitroverbindung entspricht.

Die Katalysatoren-Komponente b), d.h. das Oxidationsmittel wird bei Verwendung von Sauerstoff bzw. von Sauerstoff enthaltendem Gas so gewählt, daß der Sauerstoffanteil 0,01 bis 6,0 Vol.-%, vorzugsweise 0,1 bis 2 Vol.-% bezogen auf eingesetztes Kohlenmonoxid beträgt. Aus Sicherheitsgründen sollten 6,0 Vol.-% nicht überschritten werden. Im Falle der Verwendung von oxidierend wirkenden Metallverbindungen werden diese im allgemeinen in Mengen von 0,1 bis 100 Gew.-% vorzugsweise 5 bis 40 Gew.-% bezogen auf die eingesetzte Nitroverbindung verwendet.

Die Katalysatoren-Komponente c) liegt im Reaktionsgemisch im allgemeinen in einer Menge von 1 bis 40 vorzugsweise 4 bis 25 Mol-% bezogen auf als Ausgangsmaterial verwendete Nitroverbindung vor, wobei sich diese Angaben auf die Gesamtmenge an basischen Verbindungen nicht jedoch auf die gegebenenfalls mitzuverwendenden Salze der Formel MeX beziehen.

Die Katalysatoren-Komponente d) liegt im Reaktionsgemisch im allgemeinen in einer Menge von 1 bis 40 Mol-% vorzugsweise 4 bis 25 Mol-% bezogen auf als Ausgangsmaterial eingesetzte Nitroverbindung vor.

Das erfindungsgemäße Verfahren kann in Abwesenheit eines Lösungsmittels erfolgen, da der Alkohol als Lösungsmittel dient, jedoch kann auch ein Lösungsmittel verwendet werden. Beispiele für solche Lösungsmittel umfassen aromatische Lösungsmittel, wie Benzol, Toluol, Xylol usw., Nitrile, wie Acetonitriol, Benzonitril, usw., Sulfone, wie Sulfolan, aliphatische halogenierte Kohlenwasserstoffe, wie 1,1,2-Trichlor-1,2,2-trifluoräthan, aromatische halogenierte Kohlenwasserstoffe, wie Monochlorbenzol, Dichlorbenzol, Trichlorbenzol usw.,

Ketone, Ester und andere Lösungsmittel, wie Tetrahydrofuran, 1,4-Dioxan, 1,2-Dimethoxyäthan und ähnliches.

Die Reihenfolge der Zugabe der Ausgangsmaterialien und des Katalysatorsystems ist nicht beschränkt und kann wahlfrei je nach der Art der verwendeten Apparatur verändert werden. Beispielsweise wird eine Ausgangsmischung aus Alkohol, Selenkatalysator, Oxidationsmittel, organische Base, Amin- und/oder Harnstoff-Verbindung und organischer Nitroverbindung in einen geeigneten druckbeständigen Reaktor, wie ein Autoklav, eingeführt, worauf weiter Kohlenmonoxid unter Druck eingebracht wird, wonach unter Erwärmung gerührt wird, bis die Urethanbildungsreaktion beendet ist. Kohlenmonoxid und gegebenenfalls das Oxidationsmittel kann in den Reaktor entweder halbkontinuierlich oder kontinuierlich eingeleitet werden, während das beim Fortschreiten der Reaktion gebildete Kohlendioxid abgetrennt wird. Die Reaktion kann sowohl ansatzweise als auch halbkontinuierlich oder kontinuierlich durchgeführt werden. Das nach Beendigung der Reaktion im Überschuß anwesende Kohlenmonoxid kann durch Rezirkulation erneut verwendet werden.

Die Reaktionstemperatur wird im allgemeinen im Bereich von 80 bis 220°C, vorzugsweise 120 bis 200°C, gehalten. Obwohl die Reaktion bei höheren Reaktionstemperaturen schneller abläuft, besteht bei Temperaturen oberhalb 220°C die Neigung zu einer thermischen Zersetzung, wodurch die Ausbeute an Urethanprodukt verringert wird. Der Reaktionsdruck, d.h. der anfängliche Kohlenmonoxiddruck vor Beginn des Aufheizens, liegt im allgemeinen im Bereich von 10 bis 300 bar, vorzugsweise 20 bis 150 bar. Die Reaktionszeit hängt von der Natur und Art der verwendeten Nitroverbindung, der Reaktionstemperatur, dem Reaktionsdruck, der Art und Menge des Katalysators und der Art der Apparatur ab, jedoch liegt sie im allgemeinen im Bereich von 5 Minuten bis 6 Stunden. Nach Beendigung der Reaktion wird die Reaktionsmischung der Abkühlung überlassen bzw. abgekühlt. Nach dem Ablassen des eingefüllten Gases wird die Reaktionsmischung einer bekannten Abtrennung, wie Filtration, Destillation oder eine andere geeignete Methode, unterworfen, um das gebildete Urethan abzutrennen.

Die nach Abtrennung des Urethans zurückbleibenden Reaktionsanteile enthalten das Katalysatorsystem sowie nicht abgetrennte Urethanrestanteile. Die Wiederverwendung dieser Rückstände ist insbesondere bei kontinuierlicher Verfahrensweise von Vorteil.

Bei der Durchführung des erfindungsgemäßen Verfahrens sollte auf Wasserausschluß geachtet werden, da trotz des Zusatzes der Katalysatoren-Komponenten d) eine teilweise hydrolytische Spaltung der erfindungsgemäßen Verfahrensprodukte bei Anwesenheit von Wasser nicht ausgeschlossen werden kann.

Der erfindungswesentliche Punkt ist beim erfindungsgemäßen Verfahren in der Mitverwendung eines Oxidationsmittels zu sehen, das in Kombination mit dem erfindungsgemäßen Katalysatoren-System selbst dann eine ausgezeichnete katalytische Wirksamkeit ermöglicht, wenn eine wesentliche Verringerung der Selenmenge im Katalysatoren-System vorgenommen wird.

Im Augenblick steht für diese überraschende Wirkung dieser Verbindungen keine plausible Erklärung zur Verfügung.

Die erfindungsgemäßen Verfahrensprodukte stellen wertvolle Zwischenprodukte zur Herstellung von Schädlingsbekämpfungsmitteln oder zur Herstellung von Polyurethanen dar. Insbesondere eignen sich die erfindungsgemäßen Verfahrensprodukte als Ausgangsmaterialien zur Herstellung der entsprechenden Isocyanate bzw. Polyisocyanate durch an sich bekannte Abspaltung der Alkoholkomponente.

Die nachfolgenden Beispiele veranschaulichen die Erfindung ohne sie jedoch einzuschränken. In den Beispielen wurden alle Reaktionen in einem mit einem Rührer versehenen Autoklaven aus rostfreiem Stahl (V 4 A) durchgeführt. Die in den Beispielen angegebenen Ausbeuten wurden jeweils aus den Ergebnissen der Gaschromatographie und Flüssigkeitschromatographie errechnet.

Beispiel 1

17,22 g Nitrobenzol, 2,45 g Diazabicyclo-[2.2.2]-octan, 1,94 g Kaliumrhodanid, 0,14 g metallisches Selen, 2,66 g Anilin und 140 g abs. Äthanol wurden in einen 0,7 l Autoklaven eingebracht. Der Autoklav wurde 5 Minuten mit trockener Luft gespült, anschließend wurde Kohlenmonoxid in den Autoklaven unter Druck eingeleitet, bis der Anfangsdruck 100 bar bei Raumtemperatur erreichte. Unter Rühren wurde das Reaktionssystem auf 160°C aufgeheizt und 1 Stunde bei 160°C nachgerührt. Man ließ auf Raumtemperatur abkühlen, entspannte die Reaktionslösung, spülte mit Stickstoff und trennte vom festen Selen durch Filtration. Das erhaltene Filtrat wurde einer gaschromatographischen Analyse unterworfen, die ergab, daß das Nitrobenzol zu 90% umgewandelt war und das Filtrat 21,7 g Äthyl-N-phenylcarbamat enthielt. Vom eingebrachten Anilin blieben 0,4 g in der Reaktionslösung. Eine Gasanalyse zeigte, daß vor Reaktionsbeginn 0,8 Vol.-% Sauerstoff und nach Beendigung der Reaktion 0,3 Vol.-% Sauerstoff im Gasraum des Reaktionssystems nachzuweisen war.

Vergleichsbeispiel 1a

Beispiel 1 wurde ohne Luftspülung wiederholt mit der Maßgabe, daß das Reaktionssystem vor Reaktionsbeginn mit Stickstoff und nachfolgend mit Kohlenmonoxid gespült wurde. Nach entsprechendem Aufdrücken von 100 bar Kohlenmonoxid wurde wie in Beispiel 1 verfahren. Das Nitrobenzol war nur zu 33,7%

umgewandelt. Das Filtrat enthielt 10,2 g Äthyl-N-phenyl-carbamat und 0,5 g Anilin.

Vergleichsbeispiel 1b

Beispiel 1 wurde ohne Kaliumrhodanid wiederholt. Das Nitrobenzol war nur zu 12,8% umgesetzt. Das Filtrat enthielt 3,0 g Äthyl-N-phenylcarbamat und 1,47 g Anilin.

Vergleichsbeispiel 1c

Beispiel 1 wurde ohne Anilin wiederholt. Das Nitrobenzol war nur zu 10,5% umgewandelt. Das Filtrat enthielt 1,4 g Äthyl-N-phenylcarbamat.

Beispiel 2

17,22 g Nitrobenzol, 1,05 g 1,8-Diazobicyclo[5.4.0]-undecen-7, 0,14 g metallisches Selen, 2,66 g Anilin und 140 g abs. Äthanol wurden entsprechend Beispiel 1 mit trockener Luft gespült und zur Reaktion gebracht. Das Nitrobenzol war quantitativ umgewandelt und das Filtrat enthielt 23,3 g Äthyl-N-phenylcarbamat. Vom eingebrachten Anilin blieben 0,35 g in der Reaktionslösung.

Vergleichsbeispiel

Beispiel 2 wurde ohne Luftspülung wiederholt mit der Maßgabe, daß das Reaktionssystem vor Reaktionsbeginn mit Stickstoff und nachfolgend mit Kohlenmonoxid gespült wurde. Das Nitrobenzol war zu 79,7% umgewandelt. Das Filtrat enthielt 18,3 g Äthyl-N-phenylcarbamat und 0,53 g Anilin.

Beispiel 3

17,22 g Nitrobenzol, 1,05 g 1,8-Diazabicyclo-[5.4.0]-undecen-7, 0,14 g metallisches Selen, 2,60 g Anilin, 140 g abs. Äthanol und 2,5 g 1,4-Benzochinon wurden in einem 0,7 l Autoklaven eingebracht. Der Autoklav wurde mit Stickstoff und nachfolgend mit Kohlenmonoxid gespült. Anschließend wurde Kohlenmonoxid unter Druck in den Autoklaven geleitet, bis der Anfangsdruck 100 bar erreichte. Unter Rühren wurde auf 160°C aufgeheizt und 1 Stunde bei 160°C nachgerührt. Das Nitrobenzol war zu 91,2% umgewandelt. Das Filtrat enthielt 22,8 g Äthyl-N-phenylcarbamat und 0,58 g Anilin.

Beispiel 4

17,22 g Nitrobenzol, 2,45 g Diazabicyclo-[2.2.2]-octan, 1,62 g Kaliumcyanat, 0,14 g metallisches Selen, 2,66 g Anilin, 140 g abs. Äthanol und 2,5 g eines Metalloxidgemisches aus Eisen-III-oxid und Vandinpentoxid im Gewichtsverhältnis 11:1 wurden entsprechend Beispiel 3 zur Reaktion gebracht. Das Nitrobenzol war zu 98,9% umgewandelt. Im Filtrat befanden sich 25,0 g Äthyl-N-phenylcarbamat und 0,25 g Anilin.

Vergleichsbeispiel

Beispiel 4 wurde ohne Zusatz des Metalloxidgemisches wiederholt. Das Nitrobenzol war zu 16,4% umgewandelt. Im Filtrat lassen sich 6,2 g Äthyl-N-phenylcarbamat nachweisen.

Beispiel 5

Beispiel 5 wurde mit 2,5 g 1,4-Benzochinon an Stelle des Metalloxidgemisches wiederholt. Das Nitrobenzol war zu 78% umgewandelt. Im Filtrat lassen sich 20,1 g Äthyl-N-phenylcarbamat nachweisen.

Beispiel 6

17,22 g Nitrobenzol, 2,45 g Diazabicyclo-[2.2.2]-octan, 1,96 g Kaliumacetat, 0,14 g metallisches Selen, 2,66 g Anilin, 140 g abs. Äthanol und 2,5 g Metalloxidgemisch gemäß Beispiel 4 wurden entsprechend Beispiel 3 zur Reaktion gebracht. Das Nitrobenzol war quantitativ umgewandelt. Im Filtrat befanden sich 26,2 g Äthyl-N-phenylcarbamat und 0,2 g Anilin.

Vergleichsbeispiel

Beispiel 6 wurde ohne Zusatz des Metalloxidgemisches wiederholt. Das Nitrobenzol war zu 50,5% umgesetzt. Das Filtrat enthielt 15,0 g Äthyl-N-phenylcarbamat.

Beispiel 7

17,22 g Nitrobenzol, 1,96 g Kaliumacetat, 0,14 g metallisches Selen, 2,66 g Anilin, 140 g abs. Äthanol und 2,5 g Metalloxidgemisch gemäß Beispiel 4 wurden entsprechend Beispiel 3 zur Reaktion gebracht. Das Nitrobenzol war quantitativ umgewandelt. Im Filtrat befanden sich 24,7 g Äthyl-N-phenylcarbamat und 0,52 g Anilin.

Vergleichsbeispiel

Beispiel 7 wurde ohne Zusatz des Metalloxidgemisches wiederholt. Das Nitrobenzol zu 35,3% umgewandelt. Im Filtrat ließen sich 11,0 g Äthyl-N-phenylcarbamat nachweisen.

Beispiel 8

Beispiel 7 wurde mit 6 g N,N'-Diphenylharnstoff anstatt Anilin wiederholt. Das Nitrobenzol war quantitativ umgewandelt. Im Filtrat befanden sich 24,5 g Äthyl-N-phenylcarbamat.

Beispiel 9

Unter Veränderung des Gewichtsverhältnisses Eisen-III-oxid zu Vanidinpentoxid wie 10:1 wurde Beispiel 8 wiederholt. Das Nitrobenzol war quantitative umgewandelt. Im Filtrat befanden sich 23,2 g Äthyl-N-phenylcarbamat und 1,8 g Anilin.

Beispiel 10

25,46 g 2,4-Dinitrotoluol, 3,32 g 1,8-Diazabicyclo[5.4.0]undecen-7, 0,5 g metallisches Selen, 3,5 g 2,4-Diaminotoluol und 140 g abs. Äthanol wurden in einen 0,7 l Autoklaven eingebracht. Der Autoklav wurde 5 Minuten mit trockener Luft gespült, anschließend wurde

Kohlenmonoxid bis 100 bar Anfangsdruck unter Druck eingeleitet. Unter Rühren wurde das Reaktionssystem 1 Stunde auf 170°C gehalten. Die nach der Flüssigkeitschromatographie vorgenommene Analyse des von Selen abgetrennten Filtrats ergab eine quantitative Umsetzung des 2,4-Dinitrotoluols. Das Filtrat enthielt 24,7 g 2,4-Diäthoxycarbonylaminotoluol, 4,8 g 2-Nitro-4-äthoxycarbonylamino-toluol und 0,8 g 4-Nitro-2-äthoxycarbonylamino-toluol.

Vergleichsbeispiel

Beispiel 10 wurde ohne Luftspühlung wiederholt mit der Maßgabe, daß das Reaktionssystem vor Reaktionsbeginn mit Stickstoff und nachfolgend mit Kohlenmonoxid gespült wurde. Nach entsprechendem Aufdrücken von 100 bar Kohlenmonoxid wurde wie in Beispiel 11 verfahren. Das Filtrat enthielt 10,0 g 2,4-Diäthoxycarbonylamino-toluol, 9,9 g 2-Nitro-4-äthoxycarbonylamino-toluol und 7,7 g 4-Nitro-2-äthoxycarbonylamino-toluol.

Beispiel 11

25,46 g 2,4-Dinitrotoluol, 1,96 g Kaliumacetat, 0,5 g metallisches Selen, 3,5 g 2,4-Diaminotoluol, 140 g abs. Äthanol und 2,5 g Metalloxidgemisch gemäß Beispiel 5 wurden in einen 0,7 l Autoklaven eingebracht. Die Luft im Autoklaven wurde durch Stickstoffgas und dann durch Kohlenmonoxid ersetzt. Anchließen wurde Kohlenmonoxid unter Druck in den Autoklaven geleitet, bis der Anfangsdruck von 100 bar bei Raumtemperatur erreicht war. Das Reaktionssystem wurde unter Rühren aufgeheizt und 1 Stunde auf 170°C gehalten. Die nach der Flüssigkeitschromatographie vorgenommene Analyse des vom Selen und Metalloxidgemisch abgetrennten Filtrats ergab eine quantitative Umsetzung des 2,4-Dinitrotoluols. Das Filtrat enthielt 28,6 g 2,4-Diäthoxycarbonylaminotoluol.

Vergleichsbeispiel

Beispiel 11 wurde ohne Zusatz an Metalloxidgemisch wiederholt. Das Filtrat enthielt 19,2 g 2,4-Diäthoxycarbonylaminotoluol, 6,0 g 2-Nitro-4-äthoxycarbonylamino-toluol und 5,5 g 4-Nitro-2-äthoxycarbonylamino-toluol.

Beispiel 12

25,34 g 2,4-Dinitrotoluol, 0,7 g Kaliumacetat, 0,5 g metallisches Selen, 2,8 g 2-Amino-4-nitro-toluol, 1,4 g 4-Amino-2-nitrotoluol 140 g abs. Äthanol und 2,5 g Metalloxidgemisch gemäß Beispiel 4 wurden entsprechend 40 Minuten bei 170°C zur Reaktion gebracht. Das 2,4-Dinitrotoluol war quantitativ umgesetzt. Das Filtrat enthielt 23,8 g 2,4-Diäthoxycarbonylaminotoluol.

Vergleichsbeispiel

Beispiel 12 wurde ohne Zusatz an Metalloxidgemisch wiederholt. Die quantitative Analyse (Dünnschichtchromatographie : Kieselgel 60 F 254 der Fa. Merck, Äther/Petroläther 1:1 als Laufmittel) ergab, daß 2,4-Dinitrotoluol und die isomeren Nitro-äthoxy-carbonylaminotoluole als Hauptprodukte im Reaktionsgemisch vorlagen. 2,4-Diäthoxycarbonylaminotoluol war nur in Spuren zu erkennen. Da die Ausgangsnitroverbindung bei der Flüssigkeitschromatographie Trennprobleme verursacht, wurde auf eine quantitative Auswertung verzichtet.

Beispiel 13

25,46 g 2,4-Dinitrotoluol, 1,96 g Kaliumacetat, 0,28 g metallisches Selen, 3,5 g 2,4-Diaminotoluol, 140 g abs. Äthanol und 2,5 g eines Metalloxidgemisches aus Eisen-III-oxid und Vanadinpentoxid im Gewichtsverhältnis 1:1 wurden entsprechend Beispiel 11 umgesetzt. Das Filtrat enthielt 28,2 g 2,4-Diäthoxycarbonylaminotoluol.

Beispiel 14

Unter Veränderung des Gewichtsverhältnisses Eisen-III-oxid zu Vanadinpentoxid wie 11:1 wurde Beispiel 13 wiederholt. Das Filtrat enthält 32,5 g 2,4-Diäthoxycarbonylaminotoluol.

Vergleichsbeispiel

Beispiel 14 wurde ohne Zusatz an Metalloxidgemisch wiederholt. Das Filtrat enthielt 10,0 g 2,4-Diäthoxycarbonylaminotoluol, 7,8 g 2-Nitro-4-äthoxycarbonylamino-toluol und 10,5 g 4-Nitro-2-äthoxycarbonylamino-toluol.

**Patentanspruch**

Verfahren zur Herstellung von Urethanen durch Umsetzung von aromatischen Nitroverbindungen mit aliphatischen, cycloaliphatischen oder araliphatischen Alkoholen und Kohlenmonoxid in Gegenwart von

Selen und/oder Selenverbindungen,

aromatische Aminoverbindungen und/oder aromatische Harnstoffverbindungen, sowie

Alkalisalze schwacher Säuren und/oder bicyclische Amidine der Formel

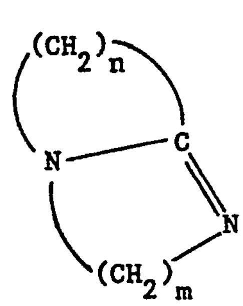

in welcher

n für eine ganze Zahl von 3 bis 5 und

m für eine ganze Zahl von 2 bis 4

stehen oder Alkalisalze schwacher Säuren in

Kombination mit sonstigen tertiären Aminen mit 3 bis 20 Kohlenstoffatomen

aufweisenden Katalysator-Systemen, dadurch gekennzeichnet, daß man Katalysator-Systeme verwendet, welche Oxydationsmittel ausgewählt aus der Gruppe bestehend aus Sauerstoff, oxydierend wirkenden, chemisch gebundenen Sauerstoff enthaltenden organischen Verbindungen und oxydierend wirkenden, chemisch gebundenen Sauerstoff enthaltenden anorganischen Verbindungen von Metallen der 1, 2, 5—8 Nebengruppe des Periodensystems der Elemente enthalten.

**Revendication**

Procédé pour la préparation d'uréthannes par réaction de composés nitrés aromatiques avec des alcools aliphatiques, cycloaliphatiques ou arylaliphatiques et le monoxyde de carbone, en présence de systèmes catalytiques contenant du sélénium et/ou des compose"s de sélénium,

des composés aminés aromatiques et/ou des urées aromatiques, ainsi que des sels alcalins d'acides faibles et/ou des amidines bicycliques de formule générale

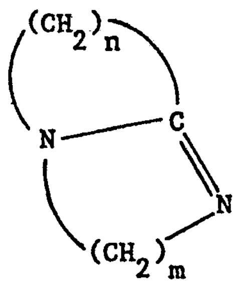

dans laquelle n est un nombre entier de 3 à 5 et m est un nombre entier de 2 à 4,

ou des sels alcalins d'acides faibles en combinaison avec d'autres amines tertiaires en $C_3$—$C_{20}$,

caractérisé en ce que l'on utilise des systèmes catalytiques qui contiennent des agents oxydants choisis parmi le gorupe constitué par l'oxygène, les composés oxydants organiques contenant de l'oxygène chimiquement lié et les composés oxydants inorganiques de métaux des sous-groupes 1, 2, 5—8 de la Classification Périodique des Eléments, contenant de l'oxygène chimiquement lié.

**Claim**

Process for the preparation of urethanes by the reaction of aromatic nitro compounds with aliphatic, cycloaliphatic or araliphatic alcohols and carbon monoxide in the presence of catalyst systems containing selenium and/or selenium compounds, aromatic amino compounds and/or aromatic urea compounds, as well as alkali metal salts of weak acids and/or bicyclic amidines of the formula

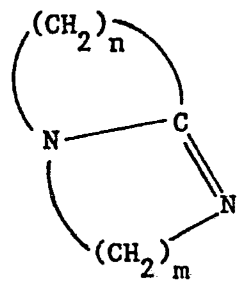

in which

n represents an integer of from 3 to 5 and

m represents an integer of from 2 to 4

or alkali metal salts of weak acids in combination with other tertiary amines having from 3 to 20 carbon atoms, characterised in that catalyst systems are used which contain oxidizing agents selected from the group consisting of oxygen, oxidizing organic compounds containing chemically bound oxygen and oxidizing inorganic compounds of metals of sub-Groups 1, 2 and 5—8 of the Periodic System of Elements containing chemically bound oxygen.